(19) Europäisches Patentamt

European Patent Office

Office européen des brevets

(11) **EP 1 693 005 A1**

(12) **EUROPEAN PATENT APPLICATION**
published in accordance with Art. 158(3) EPC

(43) Date of publication:
**23.08.2006 Bulletin 2006/34**

(51) Int Cl.:
***A61B 8/08*** (2006.01)

(21) Application number: **04820275.8**

(22) Date of filing: **09.12.2004**

(86) International application number:
**PCT/JP2004/018398**

(87) International publication number:
**WO 2005/055831 (23.06.2005 Gazette 2005/25)**

(84) Designated Contracting States:
**DE FR GB**

(30) Priority: **10.12.2003 JP 2003412384**

(71) Applicants:
• **MATSUSHITA ELECTRIC INDUSTRIAL CO., LTD.**
**Kadoma-shi, Osaka 571-8501 (JP)**
• **Kanai, Hiroshi**
**Sendai-shi,**
**Miyagi 981-0933 (JP)**

(72) Inventors:
• **KANAI, Hiroshi**
**Sendai-shi,**
**Miyagi 981-0933 (JP)**
• **HASEGAWA, Hideyuki**
**Sendai-shi,**
**Miyagi 989-3216 (JP)**

(74) Representative: **Pautex Schneider, Nicole**
**Véronique**
**Novagraaf International SA**
**25, Avenue du Pailly**
**1220 Les Avanchets - Geneva (CH)**

(54) **ULTRASONOGRAPH AND ULTRASONOGRAPHY**

(57) A new technique is disclosed for providing an ultrasonic diagnostic apparatus and an ultrasonic diagnostic method, by which it is possible to diagnose vascular endothelial function with high sensitivity through measurement of changes of elastic modulus of vascular wall in the region of tunica intima and tunica media with high precision by using ultrasonic waves in the diagnosis of vascular endothelial reaction after the stopping of avascularization. According to this technique, the apparatus comprises an arithmetic unit 19 for obtaining elastic modulus of a vascular wall 4, and the apparatus is provided with at least one of a calculation data storage unit 20 for storing changes over time of elastic modulus of vascular wall when artery is avascularized and the avascularization is then stopped, or a display unit 21 for displaying changes over time of elastic modulus of the vascular wall.

FIG. 2

**Description**

TECHNICAL FIELD

The present invention relates to an ultrasonic diagnostic apparatus for diagnosing conditions and behaviors of tissues in blood vessel wall using ultrasonic waves. The invention also relates to an ultrasonic diagnostic method.

BACKGROUND ART

In recent years, patients suffering from diseases of circulatory organs such as myocardial infarction, cerebral infarction, etc. have been increasing, and it is now an important problem to prevent and treat such diseases.
The development of myocardial infarction or cerebral infarction is deeply related with atherosclerosis. More concretely, when atheroma is formed on arterial wall or new cells of artery are not formed due to various reasons such as hypertension, artery loses elasticity and is hardened and becomes fragile. When blood vessel is blocked at a portion where atheroma is formed or when vascular tissues enclosing atheroma are ruptured and atheroma flows into blood vessel. Artery may be blocked at other portion or sclerosed portion of artery may be ruptured, thereby the diseases as described above are induced. In this respect, it is very important for the prevention and for the treatment of these diseases to diagnose atherosclerosis at earlier stage.
In the past, for the diagnosis of atherosclerotic legions, it has been practiced to directly observe and examine the conditions in blood vessel by using vascular catheter. However, to perform such diagnosis, it is necessary to insert vascular catheter into blood vessel, and this means that heavier burden is applied on the patient. For this reason, the examination using vascular catheter has been limited on the use to specify the affected site on a patient, who has been definitely determined to have such atherosclerotic lesion. For instance, this procedure has not been performed for health examination.
The measurement of cholesterol, which is one of the causes of atherosclerosis, or the measurement of blood pressure can be performed in easier manner without placing heavier load on the patients. However, these values do not directly indicate the severity of atherosclerosis.
If atherosclerosis can be diagnosed at earlier stage and therapeutic drugs to treat atherosclerosis can be administered to the patients, good effects may be provided for the treatment of atherosclerosis. However, once atherosclerosis develops and advances, it is difficult to completely recover the affected artery even though it is possible to suppress further development of atherosclerosis.
From these reasons, there are now strong demands on the development and the use of diagnostic method or diagnostic apparatus, by which it is possible to diagnose atherosclerosis in earlier stage, i.e. before atherosclerosis advances.
On the other hand, as a medical diagnostic apparatus putting fewer burdens on the patients, an ultrasonic diagnostic apparatus has been used. By irradiating ultrasonic waves from outside the body of the patient by using the ultrasonic diagnostic apparatus, it is possible to obtain information on configurations movement or quality within body can be obtained without giving pain to the patient.
In particular, if measurement is made by using ultrasonic waves, information can be obtained on movement of an object to be measured, and elastic property of the object to be measured can be determined from the displacement amount.
That is, elastic property of blood vessel inside living body can be obtained, and it is possible to directly identify how atherosclerosis has advanced. Also, the measurement can be made merely by applying ultrasonic probe on the patient, and the load on the patient is relatively low in this case. For this reason, if ultrasonic diagnostic apparatus is used, accurate diagnosis of atherosclerosis can be achieved, and the examination for preventive purpose can be performed without applying heavy burden on the patients.
However, in the ultrasonic diagnostic apparatus used in the past, as represented by the ultrasonic diagnostic apparatus used for observing the conditions and the shape of a fetus or for auscultation of heart sounds of fetus, information on shape or information on movement is not given with very high resolution. For this reason, it has not been possible to identify elastic property of artery, which expands and contracts in association with heart beat by using conventional type ultrasonic diagnostic apparatus. For example, as the apparatus disclosed in the Patent Document 1 as given below, the accuracy of the measurement on displacement of the object to be measured has not been very satisfactory.
In recent years, with rapid development of electronic technique, the measurement accuracy of the ultrasonic diagnostic apparatus has been extensively improved. In association with such advancement and progress, attempts have been made to develop an ultrasonic diagnostic apparatus, by which even slight movement of living body's tissues can be measured. For instance, in the Patent Document 2 as given below, an ultrasonic diagnostic apparatus is disclosed, by which phase tracking can be achieved with high precision by using both amplitude and phase of a detection signal and by determining instantaneous position of the object according to the least squares method with restriction. By this apparatus, it is possible to measure minute vibration of living body's tissues, which are moved by pulsation. According to the Patent Document 2, measurement can be made on minute vibration up to several hundreds of Hz on amplitude displacement in association with pulsation with amplitude of 10 mm or more with good reproducibility even when pulsation is repeated about 10 times.

The apparatus described in the Patent Document 2 can measure frequency component as high as several hundreds of Hz with good reproducibility. By converging ultrasonic beam, elastic property of the region of 1 - 2 mm in diameter on cardiac muscle or arterial wall can be measured. Also, ultrasonic signal of the component of every time phase in a single heart beat can be obtained, and it is reported that the apparatus is provided with such superior characteristics that frequency spectrum of the signal can be analyzed.

Therefore, according to the ultrasonic diagnostic apparatus using the technique of this patent publication, it is expected that progress of atherosclerosis can be determined over time without giving burden on the subject in health examination, for instance, and the disease caused by atherosclerosis can be prevented. Also, by measuring elastic property in small region of artery, it would be possible to specify a site where blood vessel rupture may possibly occur and to treat the specified site.

On inner sides of all types of blood vessels, there is endothelium, which comprises a layer of cells. The endothelial cells exhibit various types of physiological reaction in response to mechanical stress (shearing stress) caused by blood flow. One of such reactions is the production of nitrogen monoxide (NO) . NO is produced and released by NO synthetic enzyme, and it is known that NO plays a role to relax, i.e. to soften, smooth muscle in tunica media of blood vessel wall as endothelium derived relaxing factor (EDRF). The function of vascular endothelial (angioendothelial) cell is called endothelium dependent vasodilating reaction (EDR).

On the other hand, risk factors such as hypertension, hyperlipide, smoking, diabetes, etc. may decrease the functions of vascular endothelial cells. It is believed that this dysfunction represents the change of atherosclerosis in early stage. By diagnosing the functions of vascular endothelial cell, it is possible to diagnose atherosclerosis in early stage. As a method to diagnose vascular endothelial function by using EDR, a method to measure the change of diameter of artery before and after avascularization by ultrasonic waves is described in the Non-Patent Document 1 as given below. According to this method, avascularization is performed by applying cuff on brachial artery at 250 mmHg for 5 minutes. Avascularization is stopped instantaneously, and vascular diameter is intermittently measured for several dozens of seconds and vascular endothelial function is diagnosed from the increase ratio of arterial diameter.

[Patent Document 1] Japan Patent Application Publication JP-A-62-266040
[Patent Document 2] Japan Patent Application Publication JP-A-10-5226
[Non-Patent Document 1] Masayoshi HASHIMOTO and Yasuyoshi Ouchi: "Vascular Extensibility Test"; J. Japan Medical Association, Vol.120, No.8; Oct. 15, 1998, pp.S93-S96.

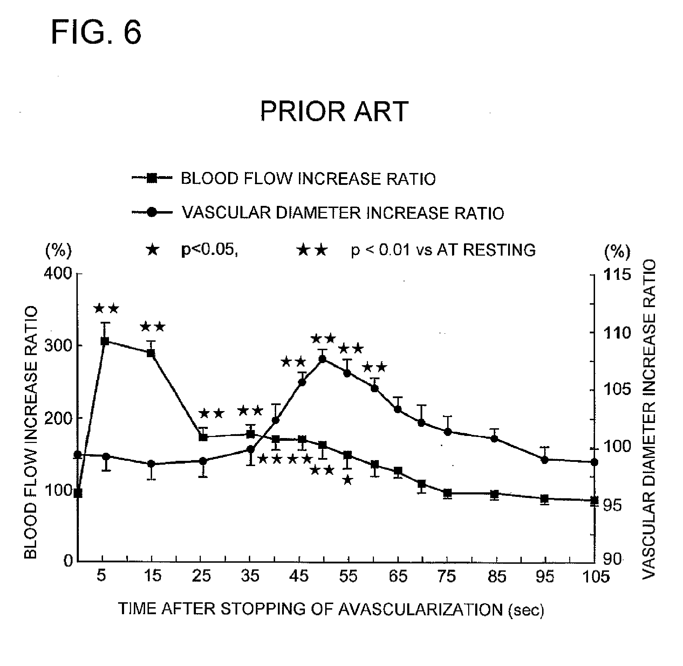

According to the method described in the Non-Patent Document 1, to measure vascular diameter, a distance between m-lines, i.e. an intermediate point between tunica media and tunica of each of the anteria wall and posterior wall, is read with precision up to 0.1 mm in cross-sectional image of the longitudinal axis of blood vessel. Then, average value is obtained from 4 to 6 measured values, and this is regarded as the measured value of arterial diameter. Fig. 6 shows the results of measurement on 9 male subjects. Black square indicates blood flow increase ratio in brachial artery after the stopping of avascularization on right forearm, and black circle indicates the ratio of the increase of the diameter of brachial artery to the vascular diameter at resting. The time after the stopping of avascularization is represented along the axis of abscissa. Blood flow increase ratio is represented on the axis of ordinate at left, and the increase ratio of vascular diameter is represented along the axis of ordinate at right. After the stopping of avascularization, blood flow ratio transiently increases, and then, it decreases over time. It is evident that, stimulated by the increase of transient increase of blood flow after the stop of avascularization, vascular diameter is significantly expanded compared with the value at resting at a time about 45 to 60 seconds after the stop of avascularization. In the results shown in Fig. 6, the increase ratio was about 6%. When the reproducibility of the value was tested after one month by this method, the increase ratio was about 10% (not shown).

According to this method, blood vessel diameter is measured up to 0.1 mm. If we consider that blood vessel diameter of brachial artery is about 3 mm, error may be as high as about 3%. That is, there is a problem in measurement accuracy in the method described in the Non-Patent Document 1.

Because this method is to measure diameter of tunica media of blood vessel, not only the thickness change of vascular wall, which is important in the diagnosis of vascular endothelial reaction, but also the value including the changes of diameter of blood vessel is measured by this method. That is, problems may also arise in the measurement sensitivity in the method described in the Non-Patent Document 1.

DISCLOSURE OF THE INVENTION

To solve the above problems, it is an object of the present invention to provide an ultrasonic diagnostic apparatus, by which it is possible to diagnose vascular endothelial function with high sensitivity by measuring the change in elastic modulus of vascular wall caused by vascular endothelial reaction after the stop of avascularization with high accuracy by using ultrasonic waves.

The ultrasonic diagnostic apparatus according to the present invention comprises an ultrasonic transmitter for transmitting ultrasonic transmission waves into tissues of living body, an ultrasonic receiver for receiving an ultrasonic echo from vascular wall in said tissues of living body, a phase detector for detecting a phase of said ultrasonic echo, an arithmetic unit for obtaining thickness change between two arbitrary positions among a plurality of positions with said vascular wall from a phase detection signal determined at said phase detector, and for obtaining elastic modulus of said vascular wall from said thickness change and a blood pressure value, and at least one of a storage unit or a display unit, said storage unit storing changes over time of elastic modulus of said vascular wall when artery is avascularized and the avascularization is then stopped, and said display unit displaying changes over time of elastic modulus of said vascular wall when artery is avascularized and the avascularization is then stopped.

With the arrangement as described above, vascular endothelial function can be diagnosed with high sensitivity.

Also, the ultrasonic diagnostic apparatus according to the present invention comprises an ultrasonic transmitter for transmitting ultrasonic transmission waves into tissues of living body, an ultrasonic receiver for receiving an ultrasonic echo from vascular wall in said tissues of living body, a phase detector for detecting a phase of said ultrasonic echo, an arithmetic unit for obtaining positional displacement of a plurality of positions within said vascular wall from a phase detection signal determined at said phase detector, obtaining thickness change between two arbitrary positions among said plurality of positions from a difference between positional changes of said two positions, and determining elastic modulus of said vascular wall from said thickness change and a blood pressure value, and at least one of a storage unit or a display unit, said storage unit storing changes over time of elastic modulus of said vascular wall when artery is avascularized and the avascularization is then stopped, and said display unit displaying changes over time of elastic modulus of said vascular wall when artery is avascularized and the avascularization is then stopped.

With the arrangement as described above, vascular endothelial function can be diagnosed with high sensitivity.

In a preferred aspect of the invention, the arithmetic unit determines elastic modulus of vascular wall, which includes at least a part of tunica media.

Also, in another preferred aspect of the invention, the arithmetic unit determines elastic modulus of vascular wall in the region of tunica intima and tunica media.

Further, the ultrasonic diagnostic method according to the present invention, comprises a transmitter/receiver for transmitting and receiving ultrasonic waves, a phase detector for detecting a phase of the received ultrasonic echo, and an arithmetic unit for calculating elastic modulus of vascular wall based on an ultrasonic echo obtained through phase detection, wherein said method comprising a step (A) of transmitting ultrasonic waves into tissues of living body including vascular wall, and receiving an ultrasonic echo obtained when said ultrasonic waves is reflected and scattered by said vascular wall, a step (B) of detecting a phase of said ultrasonic echo, a step (C) of obtaining thickness change between two arbitrary positions among a plurality of positions within said vascular wall from a phase detection signal determined by said phase detector, and determining elastic modulus of said vascular wall from said thickness change and a blood pressure value, and at least one of a step (D) of storing changes over time of elastic modulus of said vascular wall when avascularizing artery and then avascularization is stopped or a step (E) of displaying changes over time of elastic modulus of said vascular wall when avascularizing artery and then avascularization is stopped.

By this method, vascular endothelial function can be diagnosed with high sensitivity.

Also, the present invention provides an ultrasonic diagnostic method, which comprises a transmitter/receiver for transmitting and receiving ultrasonic waves, a phase detector for detecting a phase of the received ultrasonic echo, and an arithmetic unit for calculating elastic modulus of vascular wall based on an ultrasonic echo obtained through phase detection, wherein said method comprising, a step (A) of transmitting ultrasonic waves into tissues of living body including vascular wall, and receiving an ultrasonic echo obtained when said ultrasonic waves is reflected and scattered by said vascular wall, a step (B) of detecting a phase of said ultrasonic echo, a step (C) of obtaining positional displacement of a plurality of positions within said vascular wall from a phase detection signal determined by said phase detector, obtaining thickness change between two arbitrary positions among said plurality of positions from a difference of positional displacement of said two positions, and of determining elastic modulus of said vascular wall from said thickness change and a blood pressure value, and at least one of a step (D) of avascularizing artery and storing changes over time of elastic modulus of said vascular wall when avascularization is stopped or a step (E) of displaying changes over time of elastic modulus of said vascular wall.

By this method, vascular endothelial function can be diagnosed with high sensitivity.

In a preferred aspect of the invention, elastic modulus of vascular wall including at least a part of tunica media is determined in the step (C) to determine the elastic modulus.

Also, in another preferred aspect of the invention, elastic modulus of vascular wall in the region of tunica intima and tunica media is determined in the step (C) to determine the elastic modulus.

According to the present invention, the changes of elastic modulus of vascular wall caused by vascular endothelial reaction after the stopping of avascularization is measured with high accuracy by using ultrasonic waves. As a result, it is possible to provide an ultrasonic diagnostic apparatus, which can makes it possible to diagnose vascular endothelial function with high sensitivity.

BRIEF DESCRIPTION OF THE DRAWINGS

[Fig. 1] A schematical block diagram showing an arrangement of diagnosis of conditions and behaviors of tissues in vascular wall according to the present invention.
[Fig. 2] A block diagram showing an arrangement of an ultrasonic diagnostic apparatus according to the present invention.
[Fig. 3] A schematical drawing to show an embodiment of measurement of vascular endothelial reaction according to the present invention.
[Fig. 4] A schematical enlarged view of posterior vascular wall to be measured in the present invention.
[Fig. 5A] A graphic representation showing changes over time of elastic modulus in tunica intima and tunica media of vascular wall before and after the stopping of avascularization according to the present invention.
[Fig. 5B] A graphic representation showing changes over time of elastic modulus in tunica adventia of vascular wall before and after the stopping of avascularization according to the present invention.
[Fig. 6] A graphic representation showing the changes over time of blood flow increase ratio and vascular diameter increase ratio after the stopping of avascularization in conventional type measurement of vascular endothelial reaction.

BEST MODE FOR CARRYING OUT THE INVENTION

The ultrasonic diagnostic apparatus according to the present invention determines moving speed of each region of an object to be measured and amount of expansion and contraction and elastic modulus in micro-size region. The object to be measured itself does not move. In particular, the ultrasonic diagnostic apparatus of the present invention is suitable for measurement of elastic modulus in each region of living body and has high spatial resolution. For this reason, it can be preferably used for measuring expansion and contraction and elastic modulus of vascular wall. Description will be given below on a case to measure expansion and contraction and elastic modulus of vascular wall.

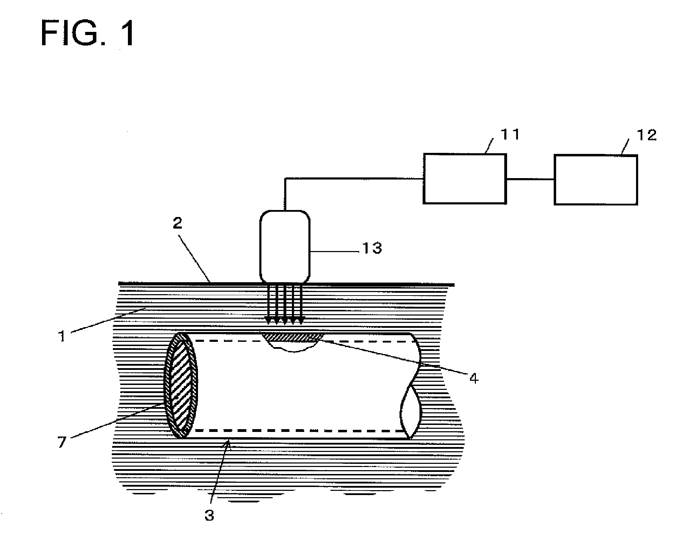

Brief description will be given below on an embodiment of the ultrasonic diagnostic apparatus according to the present invention. Fig. 1 is a schematical block diagram showing an arrangement of diagnosis of conditions and behaviors of tissues in vascular wall using the ultrasonic diagnostic apparatus 11. An ultrasonic probe 13 connected to the ultrasonic diagnostic apparatus 11 is attached on a body surface 2 of a subject under measurement, and ultrasonic waves is transmitted toward body tissues 1. The ultrasonic waves thus transmitted is reflected and scattered by a blood vessel 3. A part of the ultrasonic waves is sent back to the ultrasonic probe 13 and is received as an echo. The ultrasonic diagnostic apparatus 11 performs analysis and calculation of the received signal and determines the conditions and behaviors of a vascular wall 4. Also, a sphygmomanometer 12 is connected to the ultrasonic diagnostic apparatus 11. Blood pressure data of the subject measured by the sphygmomanometer 12 is inputted to the ultrasonic diagnostic apparatus 11. In accordance with the method disclosed in the Patent Document 2, the ultrasonic diagnostic apparatus 11 determines instantaneous position of the object to be measured by the least squares method with restriction by using both amplitude and phase of the detection signal. By carrying out phase tracking with high precision (measurement accuracy of positional change is within +0.2 micron), changes of thickness of the vascular wall 4 can be measured with sufficient accuracy. Further, by using blood pressure data obtained from the sphygmomanometer 12, elastic modulus of the vascular wall 4 can be determined.

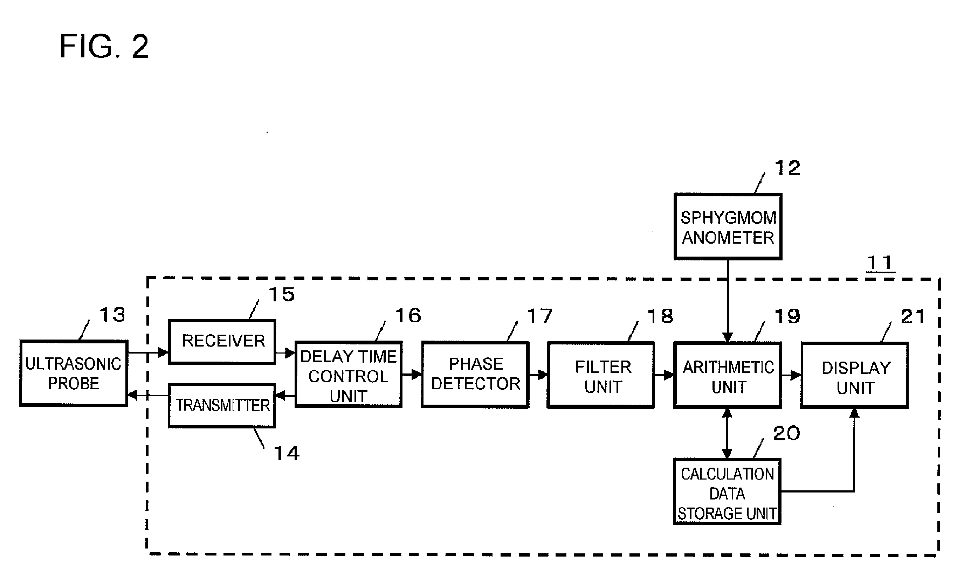

Next, detailed description will be given on the arrangement and the operation of the ultrasonic diagnostic apparatus 11 referring to the drawings. Fig. 2 is a block diagram showing an arrangement of the ultrasonic diagnostic apparatus 11. The ultrasonic diagnostic apparatus 11 comprises a transmitter 14, a receiver 15, a delay time control unit 16, a phase detector 17, a filter unit 18, an arithmetic unit 19, a calculation data storage unit 20, and a display unit 21. The transmitter 14 gives a drive pulse signal as required to the ultrasonic probe 13. Ultrasonic transmission waves as transmitted from the ultrasonic probe 13 by means of driving pulse is reflected and scattered by tissues such as the vascular wall 4, and ultrasonic reflection waves thus generated is received by the ultrasonic probe 13. The ultrasonic reflection waves received by the ultrasonic probe 13 are amplified at the receiver 15. The receiver 15 comprises an A/D converter, and the ultrasonic reflection waves amplified at the receiver 15 is converted to a digital signal.

The delay time control unit 16 is connected to the transmitter 14 and the receiver 15 and controls delay time of the drive pulse signal, which is given from the transmitter 14 to ultrasonic transducer elements of the ultrasonic probe 13. By this control, the direction of sound line and the depth of focus of the ultrasonic beam of the ultrasonic transmission waves transmitted from the ultrasonic probe 13 are changed. Also, by controlling the delay time of the ultrasonic reflection waves signal received by the ultrasonic probe 13 and amplified by the receiver 15, direction of sound line of ultrasonic waves received can be changed. Output of the delay time control unit 16 is inputted to the phase detector 17.

The phase detector 17 detects phase of the received reflection waves signal under delay control by the delay time control unit 16, and it is separated to a real part signal and a imaginary part signal. The real part signal and the imaginary part signal thus separated are inputted to the filter unit 18. The filter unit 18 removes reflection components coming from those other than the object to be measured and noise components. The phase detector 17 and the filter unit 18 may be composed of software or hardware.

Using the real part signal and the imaginary part signal of the signal after phase detection, the arithmetic unit 19 determines moving speed of a plurality of tracking positions as set within the vascular wall 4. By integrating the moving speeds, displacement in time of each of the plurality of tracking positions within the vascular wall 4 can be determined. By finding difference between two arbitrary positions among the plurality of position displacements, thickness change between the two points can be obtained. Further, from the thickness change thus obtained and from blood pressure data determined by the sphygmomanometer 12, elastic modulus of the tissues between the two points can be determined. In this case, the change in thickness between the two points may also be obtained from the phase detection signal. In the tracking method as described in the Patent Document 2, the change in relative positions of the two points, i.e. change in thickness, may be obtained from the phase detection signal without individually obtaining positional change of the two arbitrary points. The details are given, for instance, in: Hideyuki HASEGAWA, Hiroshi KANAI and Yoshiro KOIWA: "Modified Phase Tracking Method for Measurement of Change in Thickness of Arterial Wall"; J Jpn. J. Appl. Phys., Vol. 41 (2002), pp.3563-3571.

The data such as position displacement, change in thickness, elastic modulus, etc. calculated at the arithmetic unit 19 are stored at the calculation data storage unit 20 and can be read at any time as desired.

Also, the data such as position displacement, change in thickness, elastic modulus, etc. calculated at the arithmetic unit 19 are inputted to the display unit 21, and the data can be visualized. Further, if the display unit 21 is connected with the calculation data storage unit 20, various types of stored data can be displayed on the display unit 21 at any time as desired.

In this case, it is preferable that various types of data calculated at the arithmetic unit 19 are stored and displayed, while it does not matter even when the data may not be stored or may not be displayed.

Next, description will be given on the procedure to obtain elastic modulus. On a tissue, of which elastic modulus is to be calculated, maximum value and minimum value are extracted from the change in thickness within a certain period of time. Here, it is assumed that the difference between the maximum value and the minimum value is maximum thickness change $\Delta h$. Also, it is assumed that the difference between the maximum value and the minimum value of blood pressure is pulse pressure $\Delta p$. If it is supposed that thickness of the tissue to be measured is h, and that radius of blood vessel is r, the elastic modulus E can be given by the following equation:

$$E = 1/2 \ [(r/h) \ + \ 1] \ \times \ \Delta p/(-\Delta h/h)$$

Detailed description will be given below on a case where vascular endothelial reaction was measured using the ultrasonic diagnostic apparatus as describe above. Right forearm of a subject was avascularized by applying cuff at 250 mmHg for about 5 minutes. For about 120 seconds after the stopping of avascularization, the change in thickness of vascular wall was determined by the ultrasonic diagnostic apparatus. Blood pressure of the subject was continuously measured by means of tonometer. From the blood pressure data thus obtained and from thickness change, elastic modulus of vascular wall was determined, and the results of calculation were intermittently recorded for about 150 seconds before and after the stopping of avascularization. It was set that central frequency of ultrasonic waves transmitted from the ultrasonic diagnostic apparatus was 10 MHz, and that sampling frequency received was 40 MHz.

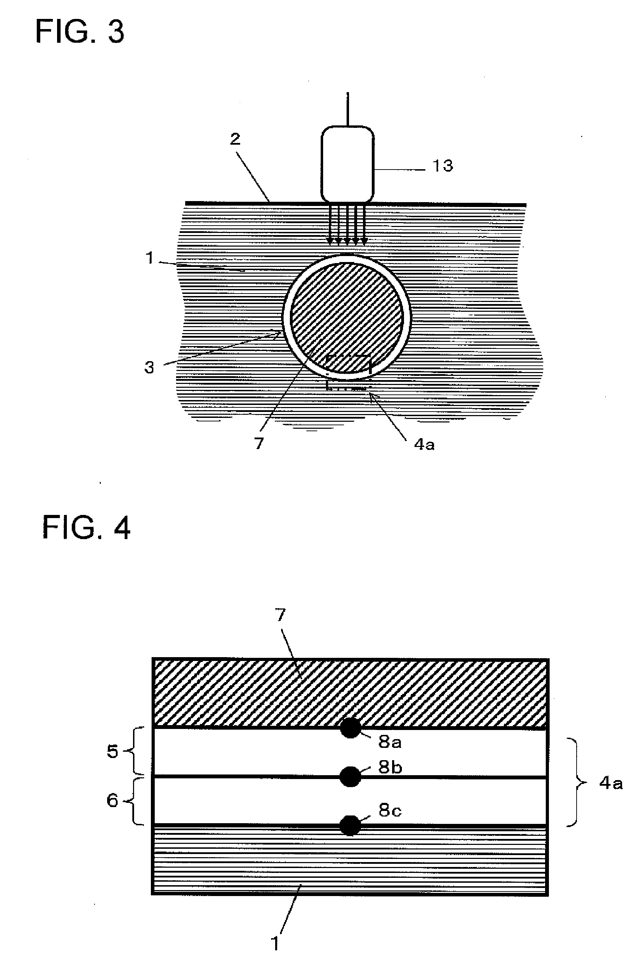

Fig. 3 is a schematical drawing of an embodiment of the measurement of vascular endothelial reaction. The ultrasonic probe 13 was set on the body surface 2 so that the blood vessel 3 can be visualized along shorter axis. The vascular wall to be measured is a posterior vascular wall 4a at the furthest position from the ultrasonic probe 13 in Fig. 3. The posterior vascular wall 4a was interposed between the blood 7 and the body tissue 1.

Fig. 4 is an enlarged view of a portion (enclosed by two-dot chain line) of the posterior vascular wall 4a in Fig. 3. The posterior vascular wall 4a is divided to a vascular wall 5 in the region of tunica intima and tunica media and a vascular wall 6 in the region of tunica adventia. Elastic modulus was to be determined for these two regions. In order to determine thickness change of each layer, tracking positions 8 were set up at three points: a boundary (8a) between the blood 7 and the posterior vascular wall 4a, a region (8b) within the posterior vascular wall 4a, and a boundary (8c) between the posterior vascular wall 4a and the body tissue 1. The thickness change of the vascular wall 5 in tunica intima and tunic media was obtained from the difference of displacement between the position 8a and the position 8b, and the thickness change of the vascular wall 6 in tunica adventia was determined from the difference of displacement between the position 8b and the position 8c. For the setting of the tracking positions, B-mode sonographic image was referred, which was obtained by using the ultrasonic probe 13.

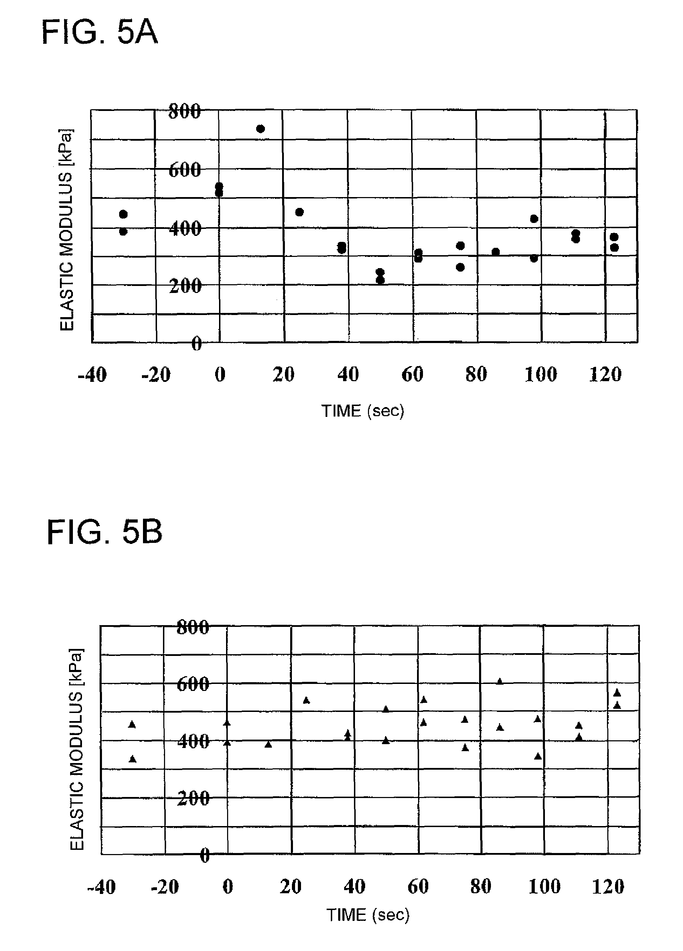

Fig. 5A and Fig. 5B each shows a graphic representation of the changes over time of elastic modulus of vascular wall before and after the stopping of avascularization. Fig. 5A shows the changes over time of elastic modulus of the vascular wall 5 in the region of tunica intima and tunic media, and Fig. 5B indicates the changes over time of elastic modulus of the vascular wall 6 in the region of tunica adventia. The time is represented along the axis of abscissa in the graph, and the time of the stop of avascularization is set to 0 [second]. In the elastic modulus of the vascular wall 6 of tunic adventia as shown in Fig. 5B, no significant change is seen for 120 seconds after the stop of avascularization. On the other hand, elastic modulus of the vascular wall 5 in the region of tunica intima and tunica media shown in Fig. 5A began to decrease immediately after the stop of avascularization (approx. 520 kPa), and it was turned to the minimum value (approx. 230 kPa) after about 50 seconds. The ratio of the change of elastic modulus in this case is about -55%.

From the results as described above, it is evident that endothelium dependent vasodilation is primarily observed on the vascular wall 5 in the region of tunica intima and tunica media, and it is evident that NO (i.e. endothelium derived relaxing factor) mainly causes reaction on the vascular wall 5 in the region of tunica intima and tunica media. Specifically, in the measurement of vascular endothelial reaction using ultrasonic waves, vascular endothelial function can be diagnosed with high sensitivity and high accuracy by taking special notice on the change of elastic modulus of the vascular wall 5 in the region of tunica intima and tunica media. This means that atherosclerosis can be diagnosed at earlier stage by the diagnosis of endothelial function as shown in the present embodiment.

In the present embodiment, the vascular wall was divided to layers of the vascular wall 5 in the region of tunica intima and tunica media and the vascular wall 6 in the region of tunica adventia, and endothelial function was diagnosed from vascular endothelial reaction when the vascular wall 5 in tunica intima and tunica media was measured. However, it is needless to say that similar diagnosis can be achieved when the vascular wall including a part of tunica media or when tunica intima and tunica media only or when tunica media only was measured because EDRF (endothelium derived relaxing factor) exerts action on smooth muscle of vascular wall in the region of tunica media.

In the present embodiment, elastic modulus was used as a notable parameter to diagnose vascular endothelial reaction, while similar diagnosis can be achieved when thickness change used in the calculation of elastic modulus or compliance (i.e. a reciprocal of elastic modulus) is used.

As described above, according to the ultrasonic diagnostic apparatus of the embodiment of the present invention, it is possible to diagnose vascular endothelial function with higher sensitivity and higher precision than in the prior art through measurement of the change of elastic modulus of the vascular wall 5 in the region of tunica intima and tunica media.

INDUSTRIAL APPLICABILITY

According to the ultrasonic diagnostic apparatus of the present invention, vascular endothelial function can be diagnosed with high sensitivity by measuring the changes of elastic modulus of the vascular wall caused by vascular endothelial reaction after the stopping of avascularization with high precision by using ultrasonic waves. Thus, this is useful as an ultrasonic diagnostic apparatus to diagnose conditions and behaviors of the tissues in vascular wall using ultrasonic waves.

**Claims**

1.  An ultrasonic diagnostic apparatus for diagnosing vascular endothelial function, said apparatus comprising:

    an ultrasonic transmitter for transmitting ultrasonic transmission waves into tissues of living body;
    an ultrasonic receiver for receiving an ultrasonic echo from vascular wall in said tissues of living body;
    a phase detector for detecting a phase of said ultrasonic echo;
    an arithmetic unit for obtaining thickness change between two arbitrary positions among a plurality of positions with said vascular wall from a phase detection signal determined at said phase detector, and for obtaining elastic modulus of said vascular wall from said thickness change and a blood pressure value; and
    at least one of a storage unit or a display unit, said storage unit storing changes over time of elastic modulus of said vascular wall when artery is avascularized and the avascularization is then stopped, and said display unit displaying changes over time of elastic modulus of said vascular wall when artery is avascularized and the avascularization is then stopped.

2.  An ultrasonic diagnostic apparatus for diagnosing vascular endothelial function said apparatus comprising:

an ultrasonic transmitter for transmitting ultrasonic transmission waves into tissues of living body;
an ultrasonic receiver for receiving an ultrasonic echo from vascular wall in said tissues of living body;
a phase detector for detecting a phase of said ultrasonic echo;
an arithmetic unit for obtaining positional displacement of a plurality of positions within said vascular wall from a phase detection signal determined at said phase detector, obtaining thickness change between two arbitrary positions among said plurality of positions from a difference between positional changes of said two positions, and determining elastic modulus of said vascular wall from said thickness change and a blood pressure value; and
at least one of a storage unit or a display unit, said storage unit storing changes over time of elastic modulus of said vascular wall when artery is avascularized and the avascularization is then stopped, and said display unit displaying changes over time of elastic modulus of said vascular wall when artery is avascularized and the avascularization is then stopped.

3. The ultrasonic diagnostic apparatus according to claim 1 or 2, wherein said arithmetic unit obtains elastic modulus of the vascular wall including at least a part of tunica media.

4. The ultrasonic diagnostic apparatus according to claim 3, wherein said arithmetic unit obtains elastic modulus of the vascular wall in tunica intima and tunica media.

5. An ultrasonic diagnostic method for diagnosing vascular endothelial function by using an ultrasonic diagnostic apparatus, comprising a transmitter/receiver for transmitting and receiving ultrasonic waves, a phase detector for detecting a phase of the received ultrasonic echo, and an arithmetic unit for calculating elastic modulus of vascular wall based on an ultrasonic echo obtained through phase detection, wherein said method comprising:

a step (A) of transmitting ultrasonic waves into tissues of living body including vascular wall, and receiving an ultrasonic echo obtained when said ultrasonic waves is reflected and scattered by said vascular wall;
a step (B) of detecting a phase of said ultrasonic echo;
a step (C) of obtaining thickness change between two arbitrary positions among a plurality of positions within said vascular wall from a phase detection signal determined by said phase detector, and determining elastic modulus of said vascular wall from said thickness change and a blood pressure value; and
at least one of a step (D) of storing changes over time of elastic modulus of said vascular wall when avascularizing artery and then avascularization is stopped or a step (E) of displaying changes over time of elastic modulus of said vascular wall when avascularizing artery and then avascularization is stopped.

6. An ultrasonic diagnostic method for diagnosing vascular endothelial function by using an ultrasonic diagnostic apparatus, comprising a transmitter/receiver for transmitting and receiving ultrasonic waves, a phase detector for detecting a phase of the received ultrasonic echo, and an arithmetic unit for calculating elastic modulus of vascular wall based on an ultrasonic echo obtained through phase detection, wherein said method comprising:

a step (A) of transmitting ultrasonic waves into tissues of living body including vascular wall, and receiving an ultrasonic echo obtained when said ultrasonic waves is reflected and scattered by said vascular wall;
a step (B) of detecting a phase of said ultrasonic echo;
a step (C) of obtaining positional displacement of a plurality of positions within said vascular wall from a phase detection signal determined by said phase detector, obtaining thickness change between two arbitrary positions among said plurality of positions from a difference of positional displacement of said two positions, and of determining elastic modulus of said vascular wall from said thickness change and a blood pressure value; and
at least one of a step (D) of storing changes over time of elastic modulus of said vascular wall when avascularizing artery and then avascularization is stopped or a step (E) of displaying changes over time of elastic modulus of said vascular wall when avascularizing artery and then avascularization is stopped.

7. The ultrasonic diagnostic method according to claim 5 or 6, wherein said step (C) of obtaining said elastic modulus is a step of obtaining elastic modulus of vascular wall including at least a part of tunica media.

8. The ultrasonic diagnostic method according to claim 7, wherein said step (C) of determining elastic modulus is a step of determining elastic modulus of vascular wall in the region of tunica intima and tunica media.

# FIG. 1

FIG. 2

EP 1 693 005 A1

## FIG. 3

## FIG. 4

FIG. 5A

FIG. 5B

# FIG. 6

## PRIOR ART

—■— BLOOD FLOW INCREASE RATIO

—●— VASCULAR DIAMETER INCREASE RATIO

★  $p < 0.05$,     ★★   $p < 0.01$ vs AT RESTING

TIME AFTER STOPPING OF AVASCULARIZATION (sec)

## INTERNATIONAL SEARCH REPORT

| | International application No. |
|---|---|
| | PCT/JP2004/018398 |

**A. CLASSIFICATION OF SUBJECT MATTER**
Int.Cl⁷ A61B8/08

According to International Patent Classification (IPC) or to both national classification and IPC

**B. FIELDS SEARCHED**

Minimum documentation searched (classification system followed by classification symbols)
Int.Cl⁷ A61B8/00

Documentation searched other than minimum documentation to the extent that such documents are included in the fields searched

| | | | |
|---|---|---|---|
| Jitsuyo Shinan Koho | 1922–1996 | Toroku Jitsuyo Shinan Koho | 1994–2005 |
| Kokai Jitsuyo Shinan Koho | 1971–2005 | Jitsuyo Shinan Toroku Koho | 1996–2005 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | Masayoshi HASHIMOTO et al., "Kekkan Shintensei Kensa", The Journal of the Japan Medical Association Tokubetsugo, 15 October, 1998 (15.10.98), Vol.120, No.8, pages S93 to S96 (particularly, page S93, right column to page S94, right column, 'Kensa no Susumekata') | 1–8 |
| Y | Edited by Motoaki SUGAWARA et al., "Ketsuryu", Kodansha Ltd., 20 June, 1985 (20.06.85), pages 275 to 281 (particularly, page 277, lines 21 to 28) | 1–8 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 23 February, 2005 (23.02.05) | 08 March, 2005 (08.03.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

14

<div style="text-align:center">**INTERNATIONAL SEARCH REPORT**</div>

| | International application No. |
|---|---|
| | PCT/JP2004/018398 |

C (Continuation).   DOCUMENTS CONSIDERED TO BE RELEVANT

| Category* | Citation of document, with indication, where appropriate, of the relevant passages | Relevant to claim No. |
|---|---|---|
| Y | Hideyuki HASEGAWA et al., "Fukin'itsu na Hekiatsu o Yusuru Kuda no Kyokusho Kabe Danseiritsu no Keisokuho", Journal of Medical Ultrasonics, 15 January, 2001 (15.01.01), Vol.28, No.1, pages J3 to J13 (particularly, page J4, '2. Hakudo ni Tomonau Domyaku Hekiatsu no Bisho Henka no Keisoku Genri', pages J7 to J8, "3.2 Domyaku ga Hekiatsu no Kin'itsu na Entokan to Minasenai Baai') | 1-8 |
| Y | Jun YOSHIDA et al., "Ninpu Dokotsu Domyaku no Naimaku Chumakuatsu to Ketsuryu Izonsei Kekkan Kakucho Hanno", Journal of Medical Ultrasonics, 15 April, 2001 (15.04.01), Vol.28, No.3, page J624 (particularly, 'Taisho to Hoho', lines 12 to 18 | 3,4,7,8 |
| Y | Naotaka SAITO et al., "Jowan Domyaku no Choonpa Keisoku ni Motozuku Kekkan Kakucho Hanno no Hyoka : Kandomyaku Byohen Suitei eno Yuyosei", Journal of Medical Ultrasonics, 15 April, 1999 (15.04.99), Vol.26, No.4, page 531 (particularly, 'Hoho to Kento Komoku', lines 1 to 5) | 1-8 |

Form PCT/ISA/210 (continuation of second sheet) (January 2004)